# EUROPEAN PATENT APPLICATION

(11) **EP 4 410 332 A1**
(43) Date of publication of application: **07.08.2024**
(21) Application number: 23154693.8
(22) Date of filing: 02.02.2023
(51) Int. Cl.: A61M 5/315

(54) **MEDICAL INJECTION DEVICE WITH AUDIBLE FEEDBACK**

(71) Applicant: Sensile Medical AG, 4600 Olten (CH)
(72) Inventor: FORSTER, Richard, 92269 Fensterbach (DE); GORSHÖFER, Andreas, 93149 Nittenau (DE); JAKOB, Michael, 93051 Regensburg (DE); PFRANG, Jürgen, 93183 Kallmünz (DE)
(74) Representative: Bardehle Pagenberg Partnerschaft mbB Patentanwälte Rechtsanwälte

(57) **Abstract**

The present disclosure relates to a medical injection device (1) comprising a housing (10) having a retaining portion (12); an actuating element (20) movably arranged in the housing (10), and a resilient element (30) movably arranged in the housing (10). In an initial state of the medical injection device (1) the resilient element (30) is pretensioned. The resilient element (30) comprises an actuating portion (32); an impacting portion (34), and an abutment portion (36) which in the initial state is in compressive engagement with the retaining portion (12). The resilient element (30) is configured to be displaced relative to the housing (10) by a displacement of the actuating element (20) against the actuating portion (32) such that the abutment portion (36) disengages from engagement with the retaining portion (12), whereby the tension of the pretensioned resilient element (30) causes the impacting portion (34) to hit an impact receiving portion (22) of the medical injection device (1), thereby generating an acoustic signal.

## Description

### 1. Technical field

The present disclosure relates to a medical injection device being configured for generating an acoustic signal.

### 2. Prior art

For medical injection devices, such as auto-injectors or injection pens, it is often necessary to provide feedback to a patient as soon as a certain operating state of the medical injection device has been reached and/or left. For example, in the case of an auto-injector that injects an emergency medication, such as adrenaline in the event of an allergic shock, the patient must be informed when the medication has been fully dispensed. Otherwise, it cannot be ensured that the injection has been interrupted, for example due to a technical malfunction. It is understood that this could result in a significant health risk for the patient.

In addition to a visual indication showing the extent to which a substance has been dispensed from the medical injection device, it has been found that acoustic feedback is often also required. The reasons for this are numerous. For example, it must be ensured that feedback is provided to visually impaired persons. In addition, an injection area may be located such that the visual indication is not in the patient's field of vision. Further, it is understood that feedback on the injection state/progress is also necessary in poor lighting conditions.

Various solutions are known from the prior art for providing acoustic feedback in a medical injection device, for example when an injection process has been completed or has begun. However, these solutions have various drawbacks as set out in the following.

First, prior art medical injection devices which are configured to provide an acoustic signal, such as those described in WO 2018/082886 A1, often include assemblies with multiple parts to generate the acoustic signal. Thereby, said multiple parts are sometimes complexly arranged. Multiple parts, particularly in a complex arrangement, increase the assembly effort and/or time. In addition, the presence of multiple parts increases the risk of incorrect assembly. Furthermore, an increased number of parts also increases the manufacturing effort as more parts must be produced. Even further, the susceptibility to failure is increased, especially if the failure of a single part leads to the failure of the medical injection device and/or the assembly which serves to generate the acoustic signal.

Second, with prior art medical injection devices which are configured to provide an acoustic signal, the unfavorable case sometimes occurs that the injection is slowed down or even interrupted by an assembly which serves to generate the acoustic signal. One reason for this may be that the assembly has a high release resistance, which slows down or even interrupts the injection process. Further, another reason may be that a resilient element of the assembly is pretensioned against another part of the medical injection device which is required to move during injection, so that the mobility of that part is impeded. A possible example for such configuration is given in Fig. 4 together with Fig. 6 of US 10,918,811 B2.

Third, the parts for generating an acoustic signal within a medical injection device regularly have only the function of generating the acoustic signal. In principle, however, it would be desirable to assign more than one function to individual parts of the medical injection device. Exemplarily to reduce the total number of parts required for the medical injection device.

Thus, it is an object of the present disclosure to provide a medical injection device that overcomes the aforementioned drawbacks at least partially.

### 3. Summary of the invention

This object is achieved, at least partly, by a medical injection device, as defined in the independent claim. Further aspects of the present disclosure are defined in the dependent claims.

In particular, the object is achieved by a medical injection device comprising a housing having a retaining portion; an actuating element movably arranged in the housing, and a resilient element movably arranged in the housing. In an initial state of the medical injection device the resilient element is pretensioned, wherein the resilient element comprises an actuating portion; an impacting portion, and an abutment portion which in the initial state is in compressive engagement with the retaining portion. The resilient element is configured to be displaced relative to the housing by a displacement of the actuating element against the actuating portion such that the abutment portion disengages from engagement with the retaining portion, whereby the tension of the pretensioned resilient element causes the impacting portion to hit an impact receiving portion of the medical injection device, thereby generating an acoustic signal.

The medical injection device may be an auto-injector, an injection pen, and/or a syringe. However, preferably the medical injection device is an auto-injector. This is as auto-injectors particularly rely thereon that the patient is informed when the medication has been fully dispensed and/or when the injection has started.

The housing may comprise a plurality of parts. For example, the housing may comprise two substantially hollow cylindrical parts that are inserted into each other after parts have been placed therein.

The retaining portion is preferably integrally formed with the housing. Particularly, the retaining portion may be integrally formed with a part of the housing that accommodates the resilient element at least partially. Hence the number of parts may be reduced, as no separate retaining portion need to be provided.

The term "resilient" as used throughout the present disclosure may refer to the ability of a material to withstand elastic deformation without deforming plastically. Hence that the resilient element is pretensioned may refer to the aspect that the resilient element is elastically deformed.

Said initial state of the medical injection device may be referred to as the state in which the medical injection device is prior to starting the injection process.

Further, the actuating portion the impacting portion, and/or the abutment portion may be delimitable sections of the resilient element. However, the actuating portion the impacting portion, and/or the abutment portion of the resilient element may also continuously transition into each other and/or overlap.

That the abutment portion is in compressive engagement with the retaining portion in the initial state may be referred to the abutment portion exerting a pressure onto the retaining portion. Further, the skilled person understands that the abutment portion being in compressive engagement with the retaining portion may serve to maintain the pretension of the resilient element in the initial state.

Particularly, the resilient element may be configured to be displaced relative to the housing by the actuating element abutting against the actuating portion when being displaced.

From the aspect wherein the tension of the pretensioned resilient element causes the impacting portion to hit the impact receiving portion the skilled person understands that the impacting portion may undergo an acceleration process.

The resilient element may be configured such that after hitting the impacting portion, the pre-tension within the resilient element is reduced to a lower amount or to zero.

The medical injection device according to the present disclosure has different advantages wherein at least two of them are described in the following in further detail.

First, compared to prior art medical injection devices the configuration of the present medical injection device allows for a reduced number of parts and/or a less complex arrangement. Accordingly, assembly effort and/or assembly time may be reduced. In addition, manufacturing effort and/or the risk of incorrect assembly can be minimized. This is particularly due to the configuration of the resilient element and the mechanically simple actuation mechanism which relies on displacing the resilient element relative to the housing.

Second, it has shown that a reduced injection and/or release resistance within the medical injection device can be achieved by means of the described configuration. Hence the risk that releasing the resilient element slows down or even interrupts the injection process can be reduced. This is particularly due to the resilient element being configured to be displaceable relative to the housing which allows for configuration whereby abrupt interruptions in movement can be avoided.

It is understood that the above-described advantages may also apply for the following in different expressions.

The resilient element may be a metal element, wherein optionally the resilient element is made of a bent sheet metal. Metal elements have proven to be advantageous as metals regularly do not creep. Further, the resilient element being made of bent sheet metal is particularly preferred as the bending allows a precise setting of the pretension which the resilient element has in the initial state.

The resilient element may have an elongated shape, wherein a longitudinal axis of the resilient element is substantially parallel to a longitudinal axis of the medical injection device. This arrangement allows a space-saving arrangement of the components. In addition, stable guidance of the resilient element within the housing can be achieved. These advantages particularly apply if the housing has a substantially hollow cylindrical shape.

The resilient element and/or the actuating element may be configured to be displaced relative to the housing along a direction which is substantially parallel to the longitudinal axis of the medical injection device, wherein optionally the resilient element and/or the actuating element are configured to be displaced relative to the housing in direction of the injection direction. By this configuration a movement of a component, e.g., of a plunger, within the medical injection device which causes the ejection of a substance may be easily used for displacing the resilient element. In this case, since no movements must be redirected, the friction but also the number of necessary parts can be reduced. Particularly the resilient element and the actuating element being configured to be displaced relative to the housing in direction of the injection direction allows for a reduced injection and/or release resistance within the medical injection device. Hence the risk that releasing the resilient element slows down or even interrupts the injection process can be reduced. The injection direction may be referred to as the direction in which a substance is ejected from the medical injection device. Further, the injection direction may be referred to as the direction in which a cannula of the medical injection device is inserted into an injection area.

The resilient element may be configured such that the abutment portion disengages from engagement with the retaining portion when the resilient element is displaced relative to the housing in direction of the injection direction. This configuration particularly allows for a reduced injection and/or release resistance within the medical injection device but also for a simple configuration of the medical injection device. Exemplarily, by this configuration a movement of a component, e.g., of a plunger, within the medical injection device which causes the ejection of a substance may be used for displacing the resilient element. Thereby no movements must be redirected so that friction but also the number of parts can be reduced.

The resilient element may comprise a bent portion which represents a first end of the resilient element. With the help of the bent portion, the bending properties of the resilient element can be specifically adjusted. Particularly, if the resilient element is made of sheet metal. In addition, the bent portion may be used to form two spring arms between which the bent portion constitutes an elastic joint. Exemplarily, one spring arm may comprise the actuating portion, whereas the other spring arm comprises the impacting portion and the abutment portion. Further, a more compact design of the resilient element can be achieved by bending. Thus, the required installation space may be reduced. Optionally in the initial state the bent portion comprises a bending angle which lies in a range from 160° to 210°, further optionally from 170° to 200°, and even further optionally from 180° to 190°. These angles have proven to a allow for a sufficient pretension within the resilient element.

The resilient element may be configured such that the abutment portion moves in a direction which is substantially perpendicular to the longitudinal axis of the medical injection device after the abutment portion disengages from engagement with the retaining portion, wherein optionally the abutment portion moves towards the longitudinal axis of the medical injection device. By the resilient element being configured such that the abutment portion moves in a direction which is substantially perpendicular to the longitudinal axis of the medical injection device after the abutment portion disengages from engagement with the retaining portion an impact on a part extending along the longitudinal axis can be achieved. This allows for better sound propagation and thus a clearer acoustic signal. Moreover, it has shown that by means of the abutment portion being configured to move towards the longitudinal axis of the medical injection device the volume of the acoustic signal may be increased. Particularly when compared to the case wherein the abutment portion is configured to move outwards from the longitudinal axis of the medical injection device. One reason for this may be that in the case of outward hitting, direct noise damping takes place because the medical injection device is enclosed by the patient's hand, whereas this is not the case in the case of inward hitting, i.e., towards the longitudinal axis of the medical injection device. Nevertheless, it is understood that in other configurations the abutment portion may move away from the longitudinal axis of the medical injection device after the abutment portion disengages from engagement with the retaining portion.

The impacting portion may be adjacent to the abutment portion. This allows the impacting portion to experience a particularly high acceleration. Thereby the acoustic signal may be provided with a high volume.

The resilient element may comprise a sliding portion which slidingly contacts the housing, wherein optionally the sliding portion is in substantially planar contact with the housing. By the resilient element slidingly contacting the housing a high release resistance, which slows down or even interrupts the injection process can be avoided. Especially by the sliding portion being in substantially planar contact with the housing a particularly reduced injection and/or release resistance within the medical injection device may be achieved.

Optionally, the impact receiving portion forms part of the actuating element. Thereby it can be achieved that the actuating element fulfils more than one function within the medical injection device. Hence, the total number of parts required for the medical injection device may be further reduced. Exemplarily no additional impact receiving part must be provided. Nevertheless, configurations are conceivable where the impact receiving portion does not form part of the actuating element, e.g., when the abutment portion moves away from the longitudinal axis of the medical injection device after the abutment portion disengages from engagement with the retaining portion.

The impacting portion may abut the impact receiving portion after hitting the impact receiving portion, optionally such that the resilient element remains under tension. Thereby it may be avoided that the resilient element is loose within the medical injection device which could create irritating acoustic signals. Further, by the resilient element being under tension after hitting the impact receiving portion a precise acoustic signal can be achieved. This is as it can be reliably avoided that due to a lack of tension the impacting portion hits several times onto the impact receiving portion.

Optionally, the actuating element is surrounded by the housing, wherein the resilient element is arranged between the actuating element and the housing. This arrangement facilitates the resilient element to be displaced relative to the housing by a displacement of the actuating element against the actuating portion such that the abutment portion disengages from engagement with the retaining portion of the housing. Further optionally the resilient element is arranged in a recess formed inside the housing. Preferably the retaining portion is thereby also arranged in the recess. Said recess provides improved guidance for the resilient element.

The housing and/or the actuating element may have a hollow shape, wherein optionally the housing and/or the actuating element have a substantially hollow cylindrical shape. These shapes allow for a particularly space-saving design, especially in connection with the technical embodiments described herein.

The actuating element may have an actuation surface for abutting the actuating portion. Exemplarily, when the actuating element has a substantially hollow cylindrical shape, the actuation surface for abutting the actuating portion may be provided on a circumferential protrusion which radially protrudes from the actuating element. Optionally in a state after injection is completed the actuation surface abuts the actuating portion and the actuating portion abuts the retaining portion. Thereby a further movement of the actuating element within the medical injection device may be prevented. Thus, it can be avoided that the actuating element unintentionally interferes with further elements of the medical injection device.

The acoustic signal may have an acoustic pressure of at least 30 dB, optionally at least 60 dB, further optionally at least 80 dB, and even further optionally at least 100 dB. These values have proven to allow for a sufficient volume of the acoustic signal for different applications. The acoustic pressure may be exemplarily influenced by the selected material(s) and/or by the selected pretension.

### 4. Brief description of the accompanying figures

In the following, the accompanying figures are briefly described:
Fig. 1 shows, a part of a medical injection device according to the present invention before generating an acoustic signal;
Fig. 2 shows a detail view of Fig. 1;
Fig. 3 shows the part of the medical injection device after generating an acoustic signal, and
Fig. 4 shows a detail view of Fig. 3.

### 5. Detailed description of the figures

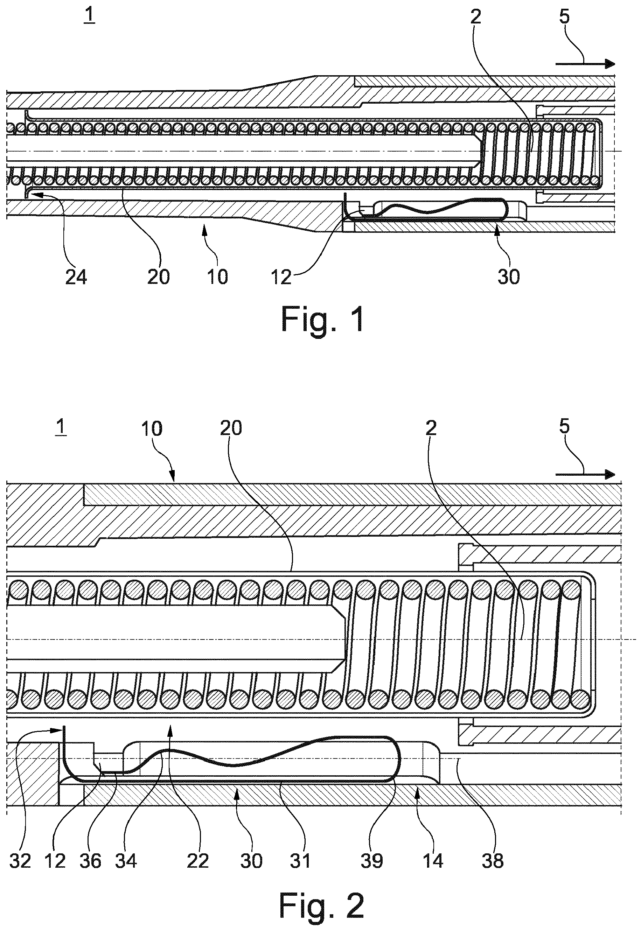

As illustrated in **Figs. 1 to 4****,** the medical injection device 1 comprises a housing 10 having a retaining portion 12; an actuating element 20 movably arranged in the housing 10, and a resilient element 30 movably arranged in the housing 10. The resilient element 30 comprises an actuating portion 32; an impacting portion 34, and an abutment portion 36. Said retaining portion 12 is integrally formed with a part of the housing 10. The medical injection device 1 as shown is configured to generate an acoustic signal when the injection process is completed, namely when a substance has been completely ejected from the medical injection device **1.**

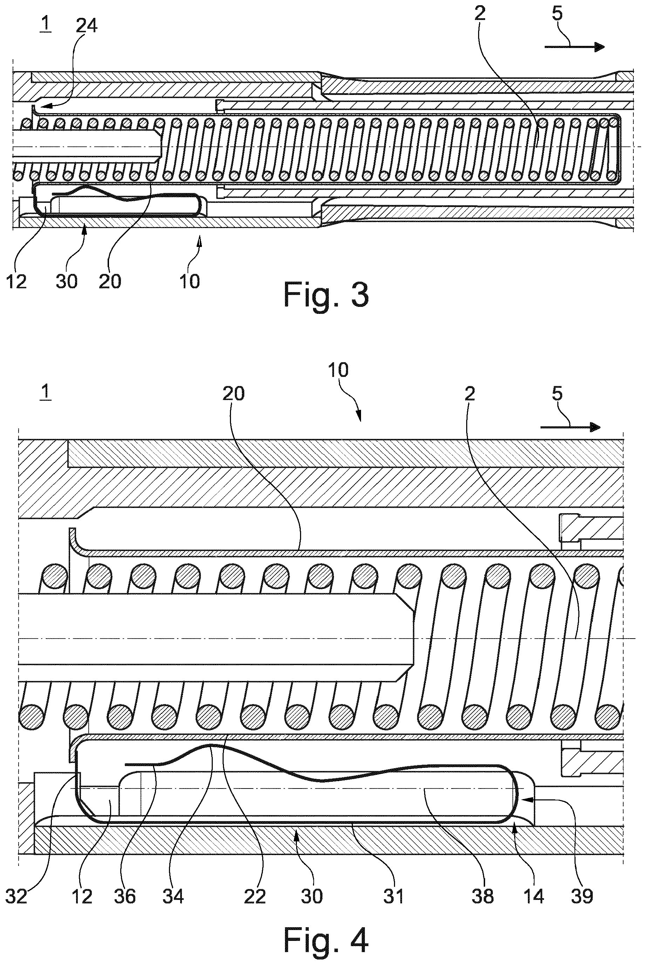

Further, even though it is not explicitly shown if the injection process has started, the skilled person takes from comparing **Fig. 2** with **Fig. 4** that in an initial state of the medical injection device 1, when the injection process has not started, the resilient element 30 is pretensioned. Particularly, the abutment portion 36 in the initial state is in compressive engagement with the retaining portion 12. Thereby the pretension of the resilient element 30 is maintained.

Moreover, as shown in detail in **Fig. 2** and **Fig. 4****,** the resilient element 30 is configured to be displaced relative to the housing 10 by a displacement of the actuating element 20 against the actuating portion 32 such that the abutment portion 36 disengages from engagement with the retaining portion 12, whereby the tension of the pretensioned resilient element 30 causes the impacting portion 34 to hit an impact receiving portion 22 of the medical injection device 1, thereby generating an acoustic signal. Said impact receiving portion 22 forms part of the actuating element 20. As illustrated, the displacement of the actuating element 20 is achieved by the relaxation of a coil spring, whereas other means to achieve said displacement are conceivable.

The resilient element 30 has an elongated shape, wherein a longitudinal axis 38 of the resilient element 30 is substantially parallel to a longitudinal axis 2 of the medical injection device 1. Further, the resilient element 30 and the actuating element 20 are configured to be displaced relative to the housing 10 along a direction which is substantially parallel to the longitudinal axis 2 of the medical injection device 1. In more detail, the resilient element 30 and the actuating element 20 are configured to be displaced relative to the housing 10 in direction of the injection direction 5. Furthermore, the resilient element 30 is configured such that the abutment portion 36 disengages from engagement with the retaining portion 12 when the resilient element 30 is displaced relative to the housing 10 in direction of the injection direction 5.

As depicted in **Fig. 2****,** the resilient element 30 comprises a bent portion 39 which represents a first end 39 of the resilient element 30, thereby in the initial state the bent portion 39 comprises a bending angle which lies in a range from 180° to 190°. Further, the resilient element 30 comprises a sliding portion 31 which slidingly contacts the housing 10, wherein the sliding portion 31 is in substantially planar contact with the housing 10. Moreover, the impacting portion 34 is adjacent to the abutment portion 36. Further, as shown in the **Figs. 1 to 4****,** the resilient element 30 is configured such that the abutment portion 36 moves in a direction which is substantially perpendicular to the longitudinal axis 2 of the medical injection device 1 after the abutment portion 36 disengages from engagement with the retaining portion 12. Particularly, the abutment portion 36 moves towards the longitudinal axis 2 of the medical injection device **1.**

As shown in **Fig. 4****,** the impacting portion 34 abuts the impact receiving portion 22 after hitting the impact receiving portion 22, such that the resilient element 30 remains under tension.

Further shown in **Fig. 4** is that the actuating element 20 has an actuation surface 24 for abutting the actuating portion 32, wherein in a state after injection is completed the actuation surface 24 abuts the actuating portion 32 and the actuating portion 32 abuts the retaining portion 12.

The actuating element 20 which has a substantially hollow cylindrical shape is surrounded by the housing 10 which also has a substantially hollow cylindrical shape. Thereby the resilient element 30 is arranged between the actuating element 20 and the housing 10, namely in a recess 14 formed inside the housing 10.

### List of reference signs

- 1: medical injection device
- 2: longitudinal axis of the medical injection device
- 5: injection direction
- 10: housing
- 12: retaining portion
- 14: recess
- 20: actuating element
- 22: impact receiving portion
- 24: actuation surface
- 30: resilient element
- 31: sliding portion
- 32: actuating portion
- 34: impacting portion
- 36: abutment portion
- 38: longitudinal axis of the resilient element
- 39: first end/bent portion

## Claims

1. A medical injection device (1) comprising
a housing (10) having a retaining portion (12);
an actuating element (20) movably arranged in the housing (10), and
a resilient element (30) movably arranged in the housing (10), wherein in an initial state of the medical injection device (1) the resilient element (30) is pretensioned, wherein the resilient element (30) comprises
an actuating portion (32);
an impacting portion (34), and
an abutment portion (36) which in the initial state is in compressive engagement with the retaining portion (12),
wherein the resilient element (30) is configured to be displaced relative to the housing (10) by a displacement of the actuating element (20) against the actuating portion (32) such that the abutment portion (36) disengages from engagement with the retaining portion (12), whereby the tension of the pretensioned resilient element (30) causes the impacting portion (34) to hit an impact receiving portion (22) of the medical injection device (1), thereby generating an acoustic signal.

2. The medical injection device (1) according to the preceding claim, wherein the resilient element (30) is a metal element, wherein optionally the resilient element (30) is made of a bent sheet metal.

3. The medical injection device (1) according to any one of the preceding claims, wherein the resilient element (30) has an elongated shape, wherein a longitudinal axis (38) of the resilient element (30) is substantially parallel to a longitudinal axis (2) of the medical injection device (1).

4. The medical injection device (1) according to any one of the preceding claims, wherein the resilient element (30) and/or the actuating element (20) are configured to be displaced relative to the housing (10) along a direction which is substantially parallel to the longitudinal axis (2) of the medical injection device (1), wherein optionally the resilient element (30) and/or the actuating element (20) are configured to be displaced relative to the housing (10) in direction of the injection direction (5).

5. The medical injection device (1) according to any one of the preceding claims, wherein the resilient element (30) is configured such that the abutment portion (36) disengages from engagement with the retaining portion (12) when the resilient element (30) is displaced relative to the housing (10) in direction of the injection direction.

6. The medical injection device (1) according to any one of the preceding claims, wherein the resilient element (30) comprises a bent portion (39) which represents a first end (39) of the resilient element (30), wherein optionally in the initial state the bent portion (39) comprises a bending angle which lies in a range from 160° to 210°, further optionally from 170° to 200°, and even further optionally from 180° to 190°.

7. The medical injection device (1) according to any one of the preceding claims, wherein the resilient element (30) is configured such that the abutment portion (36) moves in a direction which is substantially perpendicular to the longitudinal axis (2) of the medical injection device (1) after the abutment portion (36) disengages from engagement with the retaining portion (12), wherein optionally the abutment portion (36) moves towards the longitudinal axis (2) of the medical injection device (1).

8. The medical injection device (1) according to any one of the preceding claims, wherein the impacting portion (34) is adjacent to the abutment portion (36).

9. The medical injection device (1) according to any one of the preceding claims, wherein the resilient element (30) comprises a sliding portion (31) which slidingly contacts the housing (10), wherein optionally the sliding portion (31) is in substantially planar contact with the housing (10).

10. The medical injection device (1) according to any one of the preceding claims, wherein the impact receiving portion (22) forms part of the actuating element (20).

11. The medical injection device (1) according to any one of the preceding claims, wherein the impacting portion (34) abuts the impact receiving portion (22) after hitting the impact receiving portion (22), optionally such that the resilient element (30) remains under tension.

12. The medical injection device (1) according to any one of the preceding claims, wherein the actuating element (20) is surrounded by the housing (10), wherein the resilient element (30) is arranged between the actuating element (20) and the housing (10), wherein optionally the resilient element (30) is arranged in a recess (14) formed inside the housing (10).

13. The medical injection device (1) according to any one of the preceding claims, wherein the housing (10) and/or the actuating element (20) have a hollow shape, wherein optionally the housing (10) and/or the actuating element (20) have a substantially hollow cylindrical shape.

14. The medical injection device (1) according to any one of the preceding claims, wherein the actuating element (20) has an actuation surface (24) for abutting the actuating portion (32), wherein optionally in a state after injection is completed the actuation surface (24) abuts the actuating portion (32) and the actuating portion (32) abuts the retaining portion (12).

15. The medical injection device (1) according to any one of the preceding claims, wherein the acoustic signal has an acoustic pressure of at least 30 dB, optionally at least 60 dB, further optionally at least 80 dB, and even further optionally at least 100 dB.
